# EUROPEAN PATENT APPLICATION

(11) **EP 4 163 636 A1**
(43) Date of publication of application: **12.04.2023**
(21) Application number: 21201480.7
(22) Date of filing: 07.10.2021
(51) Int. Cl.: G01N 33/68

(54) **METHOD FOR PREDICTING ATOPIC DERMATITIS RELAPSE**

(71) Applicant: Beiersdorf Aktiengesellschaft, 20245 Hamburg (DE)
(72) Inventor: Al, Benjamin, 20354 Hamburg (DE); Seidel, Judith, 13507 Berlin (DE); Holzscheck, Nicholas, 22549 Hamburg (DE); Reuter, Jörn Hendrik, 24558 Henstedt-Ulzburg (DE)
(74) Representative: Haseltine Lake Kempner LLP

(57) **Abstract**

The present invention relates to a method for predicting atopic dermatitis relapse, a method for determining a suitable dermatological agent or cosmetic for preventing or delaying atopic dermatitis relapse, and the use of one or more biological protein marker(s) for predicting atopic dermatitis.

## Description

### FIELD OF THE INVENTION

The present invention discloses a method for predicting atopic dermatitis relapse, a method for determining a suitable dermatological agent or cosmetic agent for preventing or delaying atopic dermatitis relapse, and the use of one or more biological protein marker(s) for predicting atopic dermatitis.

### BACKGROUND OF THE INVENTION

Atopic dermatitis (AD) or eczema is a widespread, relapsing skin disease that is characterized by skin lesions at specific body sites, resulting in symptoms such as redness and itching. Atopic dermatitis is particularly common in children but can occur at any age.

Although there is no known cure for atopic dermatitis, there are treatments and self-care measures available to alleviate the symptoms of atopic dermatitis. Due to the rather complex nature of atopic dermatitis, however, there is no single therapeutic treatment that is suitable to treat all (or a majority) of patients suffering from atopic dermatitis. Even with the availability of a large variety of treatment options to control the symptoms of atopic dermatitis, lesions recur in most patients within weeks after cessation of treatment. However, relapse patterns differ from patient to patient.

To improve atopic dermatitis treatment, there is a need to move towards precision medicines due to the complexity of the disease. The active lesional state has been well-researched in the last few decades as can be seen, for example, in Renert-Yuval, Y., et al., Biomarkers in atopic dermatitis-a review on behalf of the International Eczema Council. J Allergy Clin Immunol, 2021. 147(4): p. 1174-1190.e1. For example, a systemic T_{H}2 skewed immune component has been found to drive the disease, resulting in blood biomarkers that characterize the active disease state.

Despite this research, factors that trigger new relapses are poorly understood and the known studies focus on patients in the active lesional state. This means that using currently known methods, a specific treatment can only be identified once the patient is suffering from the symptoms of atopic dermatitis.

There is a need to better identify the onset of atopic dermatitis to allow identification of a suitable treatment at an early stage of the relapse. This would allow therapeutic strategies to be adapted to the individual patient accordingly and intervention could occur before any clinical symptoms appear.

It is therefore desirable to provide a method for predicting atopic dermatitis relapse. In particular, it is desirable to provide a method that allows for predicting atopic dermatitis relapse in patients at an early stage of new outbreaks.

### SUMMARY OF THE INVENTION

The present invention is defined in the appended claims.

In accordance with a first aspect, there is provided a method for predicting atopic dermatitis relapse in a patient in remission, or exhibiting mild symptoms of atopic dermatitis, wherein the method comprises:
a) determining the level of one or more biological peptide marker(s) in a sample of blood of the said patient;
b) predicting atopic dermatitis relapse by comparing the level of the one or more biological protein marker(s) with empirically determined data representing a correlation between the level of said one or more protein marker(s) and atopic dermatitis relapse.

In accordance with a second aspect, there is provided a method for determining a suitable dermatological agent or cosmetic agent for preventing or delaying atopic dermatitis relapse, comprising the method of the first aspect followed by selecting an emollient to apply to the skin. An emollient generally known in the art as components of cosmetic compositions. In some cases, emollients have specific characteristics, such as lipids and waxes, which interact with lipids of the skin due to their lipophilic character. An emollient may be a liquid or cream which can be applied to the skin to improve the skin's properties, such as softness, and/or to reduce pain.

In accordance with a third aspect, there is provided a use of one or more biological protein marker(s) for predicting atopic dermatitis relapse in a patient in remission or exhibiting mild symptoms of atopic dermatitis according to the method the first aspect.

Certain embodiments of the present invention may provide one or more of the following advantages:
- desired ability to predict atopic dermatitis relapse before the active lesion state;
- desired reduction of level of suffering (including psychosocial pressure) due to delay or prevention of relapse;
- desired reduction of use of therapeutic agents associated with discomfort or side effects (such as corticosteroids or calcineurin inhibitors);desired ease of sample collection from the individual;
- desired speed of obtaining data level of biological protein markers;
- desired accuracy of prediction.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

It is understood that the following description and references to the figures concern exemplary embodiments of the present invention and shall not be limiting the scope of the claims.

### DETAILED DESCRIPTION

The present invention is based on the surprising finding that an increase in certain biological protein markers in blood can be used to predict atopic dermatitis relapse, even before the patient has exhibited any symptoms of atopic dermatitis.

The present method of prediction is targeted towards patients that have previously been diagnosed with and/or treated for atopic dermatitis. In particular, the patients according to the present method are in remission from atopic dermatitis or exhibit mild symptoms of atopic dermatitis. Patients in "remission" may also be referred to as "asymptomatic patients", i.e. patients that are not suffering any symptoms of atopic dermatitis at the time the method is carried out. As used herein, patients "exhibiting mild symptoms" of atopic dermatitis are participants with an EASI (Eczema Area and Severity Index) score of below 5.

As used herein, a "biological protein marker" is a measurable indicator of atopic dermatitis. The term "biological protein marker" may be used interchangeably with "protein marker" or "protein biomarker".

The prediction of atopic dermatitis relapse as disclosed herein is determined using the method of the first aspect of this invention. The prediction of atopic dermatitis relapse is determined from blood samples. The biological protein marker levels in the blood can be determined, for example, by a Multiplex assay. In one example the Multiplex assay used is the Bio-Plex Multiplex Immunoassay System, Bio-Rad^{®}. In some examples, the Multiplex assay is carried out according to the protocol in the manufacture's "Quick Guide" - Bio-Plex Pro^{™} Human Cytokine Screening Panel, Quick Guide" instruction manual #10000092045.

In certain embodiments, the blood may be obtained from a patient by several methods, including venipuncturing sampling and arterial sampling.

In the present invention the blood samples may comprise biological protein markers. In certain embodiments, the one or more biological protein marker(s) of the present method are independently selected from the protein markers in Table 1.

**Table 1: List of biological protein markers**

| No. | Protein |
|---|---|
| 1 | CCL21 |
| 2 | CXCL13 |
| 3 | CCL27 |
| 4 | CXCL5 |
| 5 | CCL11 |
| 6 | CCL24 |
| 7 | CCL26 |
| 8 | CX3CL1 |
| 9 | CXCL6 |
| 10 | GM-CSF |
| 11 | CXCL1 |
| 12 | CXCL2 |
| 13 | CCL1 |
| 14 | IFNγ |
| 15 | IL-1β |
| 16 | IL4 |
| 17 | IL8 |
| 18 | IL10 |
| 19 | IL16 |
| 20 | CXCL10 |
| 21 | CXCL11 |
| 22 | CCL2 |
| 23 | CCL8 |
| 24 | CCL13 |
| 25 | CCL22 |
| 26 | MIF |
| 27 | CXCL9 |
| 28 | CCL3 |
| 29 | CCL15 |
| 30 | CCL19 |
| 31 | CCL23 |
| 32 | CXCL16 |
| 33 | CXCL12 |
| 34 | CCL17 |
| 35 | CCL25 |
| 36 | TNF |
| 37 | IL9 |
| 38 | IL18 |
| 39 | M-CSF |
| 40 | CCL5 |

In certain embodiments, one or more biological protein marker(s) is/are selected from IL-4, CCL27, CCL5 and CCL8. For example, the level of one of the biological protein marker IL-4, CCL27, CCL5 or CCL8 is determined to carry out the method of prediction according to the present invention. In certain embodiments, the levels of two or more of the biological protein markers selected from IL-4, CCL27, CCL5 and CCL8 are determined to carry out the method of prediction according to the present invention. For example, two, three or four of the biological protein markers selected from IL-4, CCL27, CCL5 and CCL8 are determined to carry out the method of prediction according to the present invention.

IL-4 is the T_{H}2 cytokine which is very prominently associated with *active* atopic dermatitis and atopic dermatitis severity. CCL27 is a chemoattractant for memory T cells to the skin, which are important producers of the T_{H}2 cytokines IL-4 and IL-13. CCL5 has been reported in one study to be upregulated in spontaneously healed atopic dermatitis skin as opposed to active lesional and healthy skin. CCL8 has not previously been associated with atopic dermatitis. For all of these markers IL-4, CCL27, CCL5 or CCL8, not one of them has been associated with atopic dermatitis relapse. It would, therefore, not have been expected that one or more of these markers would be suitable for predicting atopic dermatitis relapses.

Once the level of the biological protein markers has been determined by the method disclosed herein, the levels are compared with empirically determined data. The empirically determined data represents a correlation between the said one or more biological proteins marker(s) and atopic dermatitis relapse.

The empirical data is determined by collecting blood samples from a range of individuals that had recently been affected by lesions. The serum was analysed, using the Bio-Plex Multiplex Immunoassay System, Bio-Rad^{®}, to quantifying the serum proteins of Table 1. The data for IL-4 was analyzed with Student's t test and CCL27, CCL5 and CCL8 were analyzed with Mann-Whitney U tests since only IL-4 was normally distributed. From this analysis a "cut off point" was determined for each biological protein marker. This "cut off point" distinguishes between whether a relapse occurred within a week of the test, or more than a week from the test. Using this method, the cut off points can be applied to predict whether a relapse is to happen within a week if the level for IL-4 is >= 51 pg/ml; the level for CCL27 >= 1028 pg/ml; the level for CCL5 <= 9558 pg/ml; and/or the level for CCL8 <= 69 pg/ml.

In certain embodiments, the comparison of detected levels of one or more biological protein marker(s) with the empirically determined data indicates a time to relapse. In certain embodiments the time to relapse is 7 days or less. In certain embodiments the time to relapse is more than 7 days.

In the method according to the second aspect, namely a method for determining a suitable dermatological agent or cosmetic agent for preventing or delaying atopic dermatitis relapse, the suitable dermatological agent or cosmetic agent may be any known agent that is known to alleviate the symptoms of atopic dermatitis. In some examples, the agent is an emollient which is applied to the skin.

In certain embodiments, the method may have one or more of the following effects:
- ability to predict atopic dermatitis relapse in a patient in remission from atopic dermatitis;
- ability to predict atopic dermatitis relapse in a patient exhibiting mild symptoms of atopic dermatitis;
- good accuracy of prediction of atopic dermatitis relapse;
- good reliability of correlation between level of biological protein marker(s) and empirical data;
- low cost of predicting atopic dermatitis relapse;
- ability to avoid symptoms of atopic dermatitis by applying emollient to skin based on prediction.

The foregoing description is directed to particular embodiments of the present invention for the purpose of illustrating it. It will be apparent, however, to one skilled in the art that many modifications and variations to the embodiments described herein are possible and the scope of the present invention is as defined in the appended claims.

### EXAMPLES

To illustrate and test the method according to the appended claims, the following data is provided.

Blood was taken from two patients, patient X and patient Y, who had recently experienced the symptoms of atopic dermatitis, such as an active flare, but were in remission at the timepoint of sampling. The serum from the sampled blood was analysed using the Bio-Plex Multiplex Immunoassay System, Bio-Rad^{®}. The data for IL-4, CCL27, CCL5 and CCL8 obtained was compared against the empirical data. As described above, the empirical data provides the following cut-off points to predict whether a relapse is to happen within a week of sampling. A relapse is predicted to occur within a week if IL-4 is >= 51 pg/ml; the level for CCL27 >= 1028 pg/ml; the level for CCL5 <= 9558 pg/ml; and/or the level for CCL8 <= 69 pg/ml.

The data for patient X and patient Y is provided in Table 2:

**Table 2: Examples for atopic dermatitis relapse prediction**

| | Biomarker level (in pg/ml) | | | | Predicted time of relapse | Actual time of relapse |
|---|---|---|---|---|---|---|
| | **IL4** | **CCL27** | **CCL8** | **CCL5** | | |
| patient X | 58.2 | 1209.4 | 81.6 | 8901.2 | <7 days | Day 1 |
| patient Y | 31.9 | 662.1 | 119.5 | 15142.1 | >7 days | Day 8 |

According to all marker levels patient X was predicted to relapse in week 1, and patient Y in week 2 or later. Indeed, patient X relapsed at day 1 and patient Y at day 8. This data shows that there is the present method provide a good correlation between the predicted time of relapse and the actual time of relapse. Similar results were observed in other patients.

## Claims

1. A method for predicting atopic dermatitis relapse in a patient in remission, or exhibiting mild symptoms of atopic dermatitis, wherein the method comprises:
a) determining the level of one or more biological protein marker(s) in a sample of blood of the said patient;
b) predicting atopic dermatitis relapse by comparing the level of the one or more biological protein marker(s) with empirically determined data representing a correlation between the level of said one or more biological protein marker(s) and atopic dermatitis relapse.

2. The method according to claim 1, wherein the one or more biological protein marker(s) is/are selected from Table 1.

3. The method according to claim 1 or claim 2, wherein the one or more biological protein marker(s) is/are selected from IL-4, CCL27, CCL5 and CCL8.

4. The method according to any preceding claim, wherein the comparison of detected levels of one or more biological protein marker(s) with the empirically determined data indicates a time to relapse.

5. The method according to claim 4, wherein the time to relapse is 7 days or less.

6. The method according to claim 4, wherein the time to relapse is more than 7 days.

7. The method according to any one of claims 1 to 6, wherein the sample of blood comprises whole blood, plasma and/or serum.

8. A method for determining a suitable dermatological agent or cosmetic agent or preventing or delaying atopic dermatitis relapse, comprising the method of any one of claims 1 to 7 followed by selecting an emollient to apply to the skin.

9. Use of one or more biological protein marker(s) for predicting atopic dermatitis relapse in a patient in remission, or exhibiting mild symptoms, of atopic dermatitis according to the method of any one of claims 1 to 7.

10. The use according to claim 9, wherein the one or more biological protein marker(s) is/are selected from the list in Table 1.

11. The use according to claim 9 or claim 10, wherein the one or more biological marker(s) is/are selected from IL-4, CCL27, CCL5 and CCL8.
